# EUROPEAN PATENT SPECIFICATION

(11) **EP 0 886 528 B1**
(45) Date of publication and mention of the grant of the patent: **20.10.2004**
(21) Application number: 97906937.4
(22) Date of filing: 14.03.1997
(51) Int. Cl.: A61K 39/118, A61K 39/02

(54) **USE OF COXIELLA BACTERIA TO TREAT AUTOIMMUNE DISEASE**
Verwendung Von Coxiella Bakterien zur Behandlung von Autoimmunkrankheiten
UTILISATION DE BACTERIES COXIELLA POUR TRAITER DES MALADIES AUTO-IMMUNES

(30) Priority: 14.03.1996 AU PN870396
(43) Date of publication of application: 30.12.1998
(73) Proprietor: THE AUSTRALIAN NATIONAL UNIVERSITY, Acton Australian Capital Territory 0200 (AU)
(72) Inventor: COWDEN, William, Butler, Kambah, ACT 2902 (AU); LAFFERTY, Kevin, John, Curtin, ACT 2605 (AU); GAZDA, Lawrence, Scott, Hawker, ACT 2614 (AU)
(74) Representative: Maschio, Antonio, Dr.
(86) International application number: PCT/AU1997/000161
(87) International publication number: WO 1997/033614

(56) References cited:
- AU-A- 5 960 096
- HUFNAGEL M. ET AL: "Hemophagocytosis: A complication of acute Q fever in a child" CLINICAL INFECTIOUS DISEASE, vol. 21, no. 4, October 1995, pages 1029-31, XP002097499
- STCHEPINSKY O. ET AL: "Endocardite à Coxiella burnetii sur prothèse valvulaire mécanique" ARCHIVE DES MALADIES DU COEUR ET DES VAISSEAUX, vol. 88, no. 4, April 1995, pages 511-515, XP002097500
- PIQUET P. ET AL: "Coxiella Burnetii infection of pseudoaneurysm of an aortic bypass graft with contiguous vertebral osteomyelitis" JOURNAL OF VASCULAR SURGERY, vol. 19, no. 1, January 1994, pages 165-168, XP002097782
- KANFER E. ET AL: "Q fever following bone marrow transplantation" BONE MARROW TRANSPLANTATION, vol. 3, no. 2, March 1988, pages 165-166, XP002097783
- ACTA VIRILOGICA (ENGLISH EDITION), Volume 38, No. 6, (1994), Prague, Czech Republick, Y.X. ZHANG et al., "Protective Immunity Induced by 67K Outer Membrane Protein of Phase 1 Coxiella Burnetii in Mice and Guinea Pigs", pages 327-332.
- ACTA VIRILOGICA (ENGLISH EDITION), Volume 38, No. 6, (1995), Prague, Czech Republick, E. GAJDOSOVA et al., "Immunogenicity of Coxiella Burnetii whole Cells and Their Outer Membrane Components", pages 339-344.
- CLINICAL AND EXPERIMENTAL IMMUNOLOGY, Volume 94, No. 3, (Dec. 1993), United Kingdom, A.A. IZZO et al., "Variation in Interferon-gamma Responses to Coxiella Burnetii Antigens with Lymphocytes from Vaccinated or Naturally Infected Subjects", pages 507-515.
- THE MEDICAL JOURNAL OF AUSTRALIA, Volume 160, No. 11, (6 June 1994), Australia, J.R. ACKLAND et al., "Vaccine Prophylaxis of Q Fever: A Follow-up Study of the Efficacy of Q-Vax (CSL) 1985-1990", pages 704-708.
- REVUE D'ELEVAGE ET DE MEDICINE VETERINAIRE DES PAYS TROPICAUX, Volume 46, Nos. 1-2, (1993), Paris, France, J.C. WILLIAMS et al., "Immunisation of Dogs with Q Fever Vaccines: Comparison of Phase 1, 11 and Phase 1 CMR Coxiella Burnetii Vaccines", pages 87-94.
- MIMS ANNUAL, published 1993 by MIMS AUSTRALIA, SYDNEY, AUSTRALIA, page 10-553.
- DERWENT ABSTRACT, Accession No. 95-244760/32, Class D16; & RU,C,2 026 342 (LENINGRAD EPIDEMIOLOGY MICROBIAL RESEARCH INSTITUTE), 10 January 1995.
- DERWENT ABSTRACT, Accession No. 93-098731/12, Class B04, D16; & SU,A,1 724 683 (LENINGRAD EPIDEMIOLOGY MICROBIAL RESEARCH INSTITUTE) 7 April 1992.
- RESPIRATION, Volume 62, No. 2, (1995), Basel, Switzerland, K. KAYSER et al., "Necrotizing Bronchitis, Angiitis and Anyloidosis Associated with Chronic Q Fever", pages 114-116.

## Description

The present disclosure relates generally to a method for ameliorating the adverse effects of autoimmune conditions and to therapeutic conditions useful for same. More particularly, the present disclosure provides a method for the prophylaxis and/or treatment of insulin-dependent diabetes mellitus (IDDM) by preventing, inhibiting, delaying onset of, or otherwise ameliorating the effects of the disease. The present disclosure further provides a method for the prophylaxis and/or treatment of pancreatic beta cell destruction in islet tissue transplanted into recipients. The present invention also provides therapeutic compositions useful in the prophylaxis and/or treatment of IDDM and pancreatic beta cell destruction.

Throughout this specification and the claims which follow, unless the context requires otherwise, the word "comprise", or variations such as "comprises" or "comprising", will be understood to imply the inclusion of a stated integer or group of integers but not the exclusion of any other integer or group of integers.

Bibliographic details of the publications referred to by author in this specification are collected at the end of the description.

IDDM is a debilitating, chronic cell mediated autoimmune disease characterised by lymphocytic infiltration of the pancreatic islets and lymphocyte mediated destruction of insulin-producing beta-cells (Bach 1994, Honeyman and Harrison, 1993) and is defined by the presence of hyperglycemia. Type 1 diabetes, or IDDM, occurs where an individual's own immune system destroys the cells responsible for insulin production; the beta cells residing in the islets of Langerhans of the pancreas. IDDM in humans, in the non-obese diabetic mouse (NOD mouse [Makino *et al*, 1980; Kanazawa *et al*, 1984]) and in the BB rat is an autoimmune disease associated with the development of auto-antibodies reactive to islet-associated antigens (Atkinson *et al*., 1986). Susceptibility to the development of disease both in humans and in non-human animals is under genetic control. Genes of the major histocompatibility complex (MHC), specifically those found in the class II MHC region, which control structures involved in the presentation of antigen to the cellular components of the immune system are to a large extent responsible for the disease-prone status (Davis *et al*., 1989).

In animal models, a single immunostimulation with live, infectious BCG (*Mycobacterium bovis*; Bacille Calmette-Guérin) vaccination (Yagi *et al*., 1991) or powerful immuno-adjuvants based on heat-killed *Mycobacterium tuberculosis* (or *Mycobacterium butyricum*) either early or late in the disease process, can largely suppress the development or progression of disease respectively (reviewed in Bach, 1994). Freund's Complete Adjuvant (FCA) [often referred to as complete Freund's adjuvant or CFA], which consists of a suspension of heat-killed *M. tuberculosis* (or *M. butyricum*) in mineral oil together with a surfactant is considered to be a particularly powerful immunological adjuvant. Immunostimulation with a single injection of FCA has been shown to substantially protect both NOD mice (Sadelain *et al*., 1990a; McInerney *et al*., 1991) and BB rats (Sadelain *et al*., 1990b) from developing diabetes.

Immuno-adjuvants such as bacterial lipopolysaccharide (LPS) or muramyl dipeptide (MDP) which are not based upon either live or heat-killed *Mycobacterium sp.* are not effective in preventing diabetes.

Another biological response modifier, OK-432, prepared from streptococci, when administered to NOD mice, has been shown to prevent the onset of diabetes until mice reach 24 weeks of age (Toyota *et al*., 1986); no information exists showing whether or not these animals remain free of diabetes once the therapy is discontinued. It has also been shown that the immunomodulating drug quinoline-3-carboxamide (Linomide) when given continuously in the drinking water to NOD mice, starting at a young age (5 weeks), will block the onset of diabetes until the mice are at least 40 weeks old (Gross *et al*., 1994). However, this agent is not as effective when therapy is started on mice which are 16 weeks old; in this case the cumulative incidence of diabetes is 28% by the time the mice are 42 weeks old even though the drug is given on a continuous basis (Gross *et al*., 1994).

FCA appears to induce cell-mediated protection against development of diabetes. It has, for example, been shown that spleen cells taken from NOD mice, which had been treated with FCA, were capable of suppressing the response of co-cultured control syngeneic spleen cells to mitogens (Sadelain *et al*., 1990a; McInerney *et al*., 1991). When spleen cells are taken from BCG vaccinated NOD mice and passaged into naive NOD mice, the latter are protected from developing diabetes (Harada *et al*., 1990; Yagi *et al*., 1991). The same effect has been observed in FCA-treated BB rats (Qin *et al*., 1992). It should be noted that others have found FCA to be less effective than BCG in transferring protection in this manner (Qin *et al*., 1993; Ulaeto *et al*., 1992).

Recurrence of disease in grafted tissue is a major factor which interferes with the transplantation of pancreatic islets into spontaneously diabetic NOD mice. Currently it is not possible to successfully transplant islets into animals that have developed disease spontaneously without recourse to extensive immunosuppression (Wang *et al*., 1991). Recurrence of disease can, however, be blocked in spontaneously diabetic NOD mice if they are treated with FCA (Wang *et al*., 1992; Lakey *et al*., 1992) or BCG (Lakey *et al*., 1994) prior to transplantation.

Immunostimulation of NOD mice by either BCG or FCA does not totally block the autoimmune response, instead the response is converted from a destructive into a non-destructive form of auto-immunity (Shehadeh *et al*., 1993). Thus, functionally these agents do not prevent or reverse autoimmunity directed against islet tissue, although they do prevent development of insulin-dependent diabetes.

It would appear, therefore, that deviation of the immune response away from destructive autoimmunity by immunostimulation could offer a benign approach to the prevention of diabetes and the development of more effective means for the transplantation of islets in the case of existing disease.

However, neither FCA nor BCG is an ideal therapeutic agent for preventing the development of IDDM. Despite the fact that FCA is a very powerful immuno-adjuvant, it has not found wide-spread use outside the laboratory because of the adverse tissue reaction (severe ulceration) it provokes in mammals. In most countries, FCA is banned from vetermary use and, of course, it cannot be used in humans. BCG is an infectious agent which cannot be given to certain individuals, especially those who are immunosuppressed.

In work leading to the present invention, the inventors sought to develop agents capable of blocking the development of IDDM in humans which are sufficiently benign for general use and most effective in terms of deviating the immune response away from destructive autoimmunity.

Accordingly, one aspect of the present invention contemplates a use of a species of *Coxiella* which has been rendered non-infectious in the manufacture of a medicament for use in the treatment of an autoimmune disease.

Another aspect of the present invention provides a use of a species of *Coxiella* which has been rendered non-infectious in the manufacture of a medicament for use in prolonging survival of islet tissue transplanted into a mammal.

Suitably the medicament provides a therapeutic composition for use in preventing, inhibiting, delaying onset of or otherwise ameliorating the effects of an autoimmune disease in a mammal said composition comprising a species of *Coxiella* or analogous or homologous components thereof and one or more pharmaceutically acceptable carriers and/or diluents.

*Coxiella* is a genus of Gram negative bacteria of the Rickettsieae. The only known species to date is *Coxiella burnetii* which is the causative agent of Q fever in man. Reference herein to "*Coxiella*" species, therefore includes *C*. *burnetii*. The present invention requires that the *Coxiella* species be attenuated, killed or otherwise rendered non-infectious prior to use. Immunogenicity of *Coxiella burnetii* either as whole cells or components thereof has previously been described (Zhang et al. Acta Virologica 38,327-332 (1994), Gajdosova et al. Acta Virologica 38; 339-344 (1994) and Ackland et al The Medical Journal of Australia Vol. 160; p. 704-709 (1994)).

The use of non-infectious agents in the prophylaxis and/or treatment of autoimmune disease conditions offers a major advantage in clinical terms. Infectious agents, even those such as BCG, frequently pose a problem if administered to an immunocompromised individual and, while FCA might be effective in humans, its use is precluded by the severe ulcerative skin lesions which develop following its use. In accordance with the present invention, an ideal agent would be capable of blocking the induction of the autoimmune disease as well as inhibiting disease recurrence in transplanted islet tissue in diabetic patients.

The present invention is particularly directed and exemplified with reference to IDDM as the autoimmune disease. This is done, however, with the understanding that the present invention extends to a range of autoimmune conditions and in particular, autoimmune conditions which can be treated by deviating an immune response away from destructive autoimmunity. Examples of autoimmune conditions contemplated herein other than IDDM include but are not limited to pernicious anaemia, autoimmune chronic hepatitis, ulcerative colitis, primary biliary cirrhosis multiple sclerosis and systemic lupus erythematosis (SLE).

According to a preferred embodiment, the use of the present invention contemplates a method of preventing, inhibiting, delaying onset of or otherwise ameliorating the effects of IDDM in a mammal, said method comprising administering to said mammal an autoimmune-preventing effective amount of a species of *Coxiella* or analogous or homologous components thereof.

The present invention extends to the prophylaxis or treatment of any mammal such as but not limited to a human, primate, livestock animal (eg. sheep, cow, horse, pig, donkey), companion animal (eg. dog, cat), laboratory test animal (eg. mouse, rat, guinea pig, rabbit, hamster) or captive wild animal (eg. fox, deer, kangaroo).

Accordingly, a preferred aspect of the use of the present invention is directed to a method of preventing, inhibiting, delaying onset of or otherwise ameliorating the effects of IDDM in a human, said method comprising administering to said mammal an autoimmune-preventing effective amount of a species of *Coxiella* or analogous or homologous components thereof.

The present invention particularly relates to *C*. *burnetii*. A particularly useful form of *C*. *burnetii* is a killed preparation of the bacterium such as a heat killed or formulin killed preparation. One such useful form is QVAX which is a Q fever vaccine or Q fever complement fixing antigen phase I (QFA) [both available from CSL Limited, Melbourne, Australia]. The present invention extends however to live, attenuated strains of *C. burnetii* or to preparations of the bacterium killed or rendered non-infectious by other means.

The present invention further contemplates antigenic components of *C*. *burnetii* such as a lysed preparation of the whole organism, a membrane/wall preparation, an endospore preparation or one or more purified or partially purified antigenic molecules therefrom or analogous or homologous components thereof.

Accordingly, a particularly preferred embodiment of the use of the present invention is directed to a method for preventing, inhibiting, delaying onset of or otherwise ameliorating the effects of IDDM or pancreatic beta cell destruction in islet tissue transplantation recipients in a human, said method comprising administering to said human, an effective amount of killed or attenuated *C*. *burnetii* or an antigenic component thereof or analogous or homologous components thereof.

Preferably, QVAX or QFA is administered.

Generally, administration is for a time and under conditions sufficient for the immune response to deviate from a destructive immune response to a non-destructive immune response.

This and other aspects of the present invention are predicted in part of the surprising result that a Q fever antigen (*C. burnetii*), and in particular killed *C. burnetii* in the form of, for example, QFA or QVAX when injected into NOD mice inhibits diabetes in a high percentage (≥90%) of treated mice. The agents of the present invention are not recognised immuno-adjuvants and yet are as effective as either BCG or FCA in preventing the development of diabetes in NOD mice.

The present disclosure contemplates *Coxiella* sp and in particular *C. burnetii* and antigenic components thereof as well as recombinant or synthetic forms of the antigenic components and analogues or homologues thereof from another source. The latter molecules may be identified, for example, by natural product screening of coral, ocean beds, plants, soil and other microorganisms. These molecules may structurally mimic the antigenic components from *C*. *burnetii* or may only functionally mimic these components by preventing, inhibiting, delaying onset of, curing or otherwise ameliorating the effects of an autoimmune disease and in particular IDDM. One convenient way of screening for analogues or homologues is *via* anti-idiotypic antibody screening. For example, antibodies are raised to particular antigenic components of *C*. *burnetii* and anti-idiotypic antibodies raised to the first mentioned antibodies. The anti-idiotypic antibodies are then used to screen for molecules capable of binding or otherwise interacting with these antibodies.

Alternatively, chemical analogues may be made from isolated antigenic components of *C. burnetii* or from analogous or homologous components thereof.

Analogues of *C*. *burnetii* antigenic components contemplated herein include, but are not limited to, where the antigenic component is proteinaceous, modifications to side chains, incorporation of unnatural amino acids and/or their derivatives during peptide, polypeptide or protein synthesis and the use of crosslinkers and other methods which impose conformational constraints on the proteinaceous molecule or their analogues. Such analogues are particularly useful if they are more stable when used for administration in a vaccine formulation.

Examples of side chain modifications contemplated by the present disclosure include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation *via* O-acylisourea formation followed by subsequent derivitisation, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, omithine, sarcosine, 4-amino-3-hydroxy-6-methylheptanoic acid, 2-thienyl alanine and/or D-isomers of amino acids. A list of unnatural amino acid, contemplated herein is shown in Table 1.

Crosslinkers can be used, for example, to stabilise 3D conformations, using homo-bifunctional crosslinkers such as the bifunctional imido esters having (CH₂)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH). In addition, peptides can be conformationally constrained by, for example, incorporation of C_{α} and N_{α}-methylamino acids, introduction of double bonds between C_{α} and C_{β} atoms of amino acids and the formation of cyclic peptides or analogues by introducing covalent bonds such as forming an amide bond between the N and C termini, between two side chains or between a side chain and the N or C terminus.

The present disclosure further contemplates chemical analogues of *C*. *burnetii* antigenic components capable of acting as antagonists or agonists of same or which can act as functional analogues of the antigenic components. Chemical analogues may not necessarily be derived from *C. burnetii* molecule but may share certain conformational similarities. Alternatively, chemical analogues may be specifically designed to mimic certain physiochemical properties of the antigenic components. Chemical analogues may be chemically synthesised or may be detected following, for example, natural product screening.

Antagonists may be important molecules to control the extent of immunostimulation or to prevent unwanted immune responses such as hypersensitivity reactions. Agonists may be important to enhance immunostimulatory properties.

**TABLE 1**

| Non-conventional amino acid | Code | Non-conventional amino acid | Code |
|---|---|---|---|
| α-aminobutyric acid | Abu | L-N-methytalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | Nmgln |
| carboxylate | | L-N-methylglutamic acid | Nmglu |
| cyclohexylalanine | | Chexa L-N-methylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmom |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Nom |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmorn | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgin | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methytcyclohexylalanine | Nmchexa | D-N-methytmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glyciae | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(*p*-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-*t*-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mava | L-α-methylornithine | Morn |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalanine | Nmhphe |
| N-(N-(2,2-diphenylethyl) | Nnbhm | N-(N-(3,3-diphenylpropyl) | Nnbhe |
| carbamylmethyl)glycine | | carbamylmethyl)glycine | |
| 1-carboxy-1-(2,2-diphenyl- | Nmbc | | |
| ethylamino)cyclopropane | | | |

In a preferred embodiment of the present invention, the human targeted for therapy would be known to be likely to develop IDDM or is a patient who has recently been diagnosed to be in the early stages of IDDM or is a patient suffering from IDDM and who could be transplanted with islet tissue from a non-diabetic donor. The human may also be an "at risk" individual due to certain environmental conditions or may have a genetic propensity to develop IDDM such as due to a family history of the disease.

Another aspect of the use of the present invention contemplates a method for prolonging survival of islet tissue transplanted into a human, said method comprising administering into said mammal an effective amount of a species of *C*. *burnetii* or analogous or homologous components thereof.

In yet another aspect the use of the present invention provides a therapeutic composition for use in preventing, inhibiting, delaying onset of or otherwise ameliorating the effects of an autoimmune disease in a mammal said composition comprising a species of *Coxiella* or analogous or homologous components thereof and one or more pharmaceutically acceptable caniers and/or diluents.

Preferably, the mammal is a human. Preferably the autoimmune condition is IDDM or pancreatic beta cell destruction in islet tissue of transplantation recipients. Preferably the *Coxiella* species is *C*. *burnetii* or an antigenic component or an analogous or homologous component thereof or is a killed or otherwise non-infectious in attenuated form of the bacterium. Most preferably, the administered agent is QFA or QVAX or functionally equivalent forms thereof.

The term "active component" is hereinafter used to refer to a *Coxiella* species and in particular *C. burnetii* or to antigenic components therefrom or analogous or homologous components thereof.

The active component is administered in therapeutically effective amounts. A therapeutically effective amount means that amount necessary at least partly to attain the desired effect, or to inhibit or delay the onset of, inhibit the progression of, or halt or prevent altogether, the onset or progression of the particular condition being treated such as IDDM. Such amounts will depend, of course, on the particular condition being treated, the severity of the condition and individual patient parameters including age, physical condition, size, weight and concurrent treatment. These factors are well known to those of ordinary skill in the art and can be addressed with no more than routine experimentation. It is preferred generally that a maximum dose be used, that is, the highest safe dose according to sound medical judgement. It will be understood by those of ordinary skill in the art, however, that a lower dose or tolerable dose may be administered for medical reasons, psychological reasons or for virtually any other reasons.

The formulation of such therapeutic compositions is well known to persons skilled in this field. Suitable pharmaceutically acceptable carriers and/or diluents include any and all conventional solvents, dispersion media, fillers, solid carriers, aqueous solutions, coatings, antibacterial and antifungal agents, isotonic and absorption delaying agents, and the like. The use of such media and agents for pharmaceutically active substances is well known in the art, and it is described, by way of example, in *Remington's Pharmaceutical Sciences*, 18th Edition, Mack Publishing Company, Pennsylvania, USA. Except insofar as any conventional media or agent is incompatible with the active ingredient, use thereof in the pharmaceutical compositions of the present disclosure is contemplated.

Therapeutic formulations suitable for injectable use include sterile aqueous solutions (where water soluble) or dispersions and sterile powders for the extemporaneous preparation of sterile injectable solutions or dispersions. The present disclosure also contemplates administration via topically applied compositions where active components may be modified to permit entry via the skin. It must be stable under the conditions of manufacture and storage and must be preserved against the contaminating action of microorganisms such as bacteria and fungi.

The carrier can be a solvent or dispersion medium containing, for example, water, ethanol, polyol (for example, glycerol, propylene glycol and liquid polyethylene glycol and the like), suitable mixtures thereof and vegetable oils. The proper fluidity can be maintained, for example, by the use of a coating such as licithin, by the maintenance of the required particle size in the case of dispersion and by the use of surfactants. The preventions of the action of microorganisms can be brought about by various antibacterial and antifungal agents, for example, parabens, chlorobutanol, phenol, sorbic acid, thirmerosal and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active components in the required amount in the appropriate solvent with various of the other ingredients enumerated above. Generally, dispersions are prepared by incorporating the various active components, sterilized where appropriate, into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient.

Preferred compositions or preparations according to the present disclosure contains between about 0.1 µg and about 2000 mg of active component. Other ranges contemplated herein include from about 1µg to about 1000 mg, from about 10 µg to about 100 mg and from about 100 µg to about 50 mg. Where killed or attenuated or otherwise non-infectious organisms are administered, a suitable range is from about 10² to about 10⁶ cells/ml of therapeutic composition being administered to a patient. The present disclosure also contemplates amounts outside this range, the important feature being an amount which is effective to induce a deviation from destructive autoimmunity to a non-destructive form of autoimmunity.

Pharmaceutically acceptable carriers and/or diluents include any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active component, use thereof in the therapeutic compositions is contemplated. Supplementary active components can also be incorporated into the compositions. Examples of supplementary components include various cytokines, insulin, antibacterial agents and immune potentiating molecules.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the disclosure are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active component is compounded for convenient and effective administration in effective amounts with a suitable pharmaceutically acceptable carrier in dosage unit form as hereinbefore disclosed. A unit dosage form can, for example, contain the principal active component in amounts ranging from about 0.1 µg to about 2000 mg. Expressed in proportions, the active component is generally present in from about 0.5 µg to about 2000 mg/ml of carrier. Expressed in cellular terms, the unit dosage may range from 10² to 10¹⁶ cells/ml of carrier or diluent. In the case of compositions containing supplementary active component, the dosages are determined by reference to the usual dose and manner of administration of the said components. The present disclosure also contemplates dosage ranges outside these amounts.

The active components of the present disclosure may be administered alone or in combination with other therapeutic molecules such as molecules which reduce effects of the autoimmune pathology associated with IDDM. A single dose may be administered and is preferred although multiple doses may be required with intervals of from minutes to hours, days to weeks or months to years.

Reference herein to "preventing" IDDM includes total prevention of IDDM or substantial prevention for a limited time or even substantially indefinitely or delaying onset of IDDM or reducing the severity or otherwise ameliorating the effects of IDDM. For example, in humans, prevention includes but is not limited to delaying onset for from 1 to 60 years or from 2 to 30 years or from 3 to 15 years. As a test system for prevention in mice may range from weeks to years.

It has been shown that the active components of the present invention including one or more Q fever antigens and in particular killed *C. burnetii* are more efficacious than either FCA or BCG in protecting beta cells from autoimmune destruction in NOD mice. Thus, NOD mice treated with doses of either FCA, BCG or Q fever antigen(s), did not develop IDDM. Significantly, more viable beta cells were found in the pancreases of mice which had been treated with the active components than in the mice treated with either FCA or BCG.

NOD female mice between 120 and 180 days of age, which had become diabetic spontaneously, treated with an active component of the present invention and then transplanted with syngeneic islet tissue essentially as described in Bowen *et al*, (1980), maintained normal blood glucose levels. Control mice, treated with saline instead of an active component and then similarly transplanted with syngeneic islet tissue, become diabetic, for example within about 10 days of tissue transplantation. Histological sections of transplanted tissue taken from the saline treated group, at the time they become diabetic, show a massive inflammatory infiltrate into the transplanted tissue, with essentially complete beta cell destruction. Histological sections taken for example one month after treatment from the animals treated with an active component show little or no inflammatory infiltrate and the beta cells are intact.

The present invention which is defined in the appended claims is further described by reference to the following non-limiting Figures and Examples. It is to be understood, however, that the Examples are included solely for the purposes of exemplifying the present invention, and should not be understood in any way as a restriction on the broader aspects of the subject invention as set out above.

In the Figures:
**Figure 1** is a graphical representation showing the prevention of IDDM in NOD mice following administration of QFA:
   - □: Saline (12/16 diabetic)
   - ◇: Saline (8/13 diabetic)
   - **•**: FCA (2/17 diabetic)
   - ■: QFA 200µl (1/14 diabetic)
**Figure 2** is a graphical representation showing the prevention of IDDM in NOD mice following administration of QFA:
   - □: Saline (8/13 diabetic)
   - •: QFA 70 µl (2/15 diabetic)
   - ■: QFA 200µl (1/14 diabetic)

In the following examples, all specific pathogen-free NOD mice were obtained from the Animal Breeding Establishment, The Australian National University, Canberra, ACT, Australia. Mice were housed under specific pathogen-free conditions with sterilised micro-isolator cages and bedding. Sterilised food and water were provided *ad libitum*. Cages were changed in laminar flow hoods using sterile technique by a laboratory assistant who was dedicated solely to this task and who was not responsible for the maintenance of any other animals. Cages were housed in hepa filtered ventilation racks with a 12 hour day night cycle. Routine serological monitoring was performed by the Murine Virus Monitoring Service, (Primary Industries, Adelaide, South Australia), for evidence of infection by any of 15 different murine viruses. Since the implementation of this procedure, the NOD mouse colony used in this study has tested negative for all of these viruses.

### EXAMPLE 1

This example demonstrates the effectiveness of a single 200 µl dose of Q fever complement fixing antigen Phase I (QFA) in inhibiting the occurrence of IDDM in NOD mice.

Female NOD littermates between 64 and 69 days of age were divided into two groups and injected intraperitoneally (ip) with either 200 µl of Q fever complement fixing antigen phase I (obtained from CSL, Melbourne Australia) or 200 µl of saline solution. Fifteen mice were injected with QFA while 14 littermates received saline solution. One animal from each group was found dead of unknown causes during the course of the experiment.

For comparison, in a similar manner, 17 female NOD littermates between the ages of 64 and 85 days of age were injected in the right rear footpad with 50 µl complete Freund's adjuvant (FCA) (Bacto lot # 784560) given as an emulsion in an equal volume of normal saline; 16 litter mates received saline solution only.

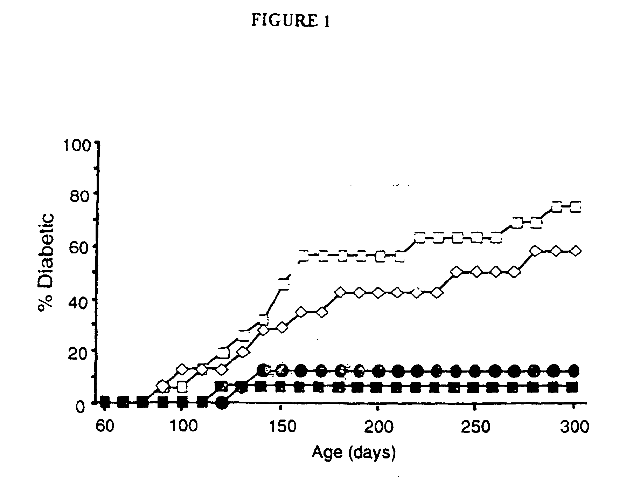

Mice in all of the four groups were checked three times per week for elevated glucose concentrations in their urine (Tes-Tape, Eli Lilly and Co, Indianapolis, IN, USA). When mice were found to have raised urinary glucose levels, whole blood glucose levels were determined with a Companion 2 Sensor (Medisense, Cambridge, MA, USA). Where two consecutive daily blood glucose readings above the 95 % confidence interval for NOD mice in this colony (3.9-9.1 mmol/L) were obtained, the animal was deemed to be diabetic. Animals were monitored until they reached 300 days of age or became diabetic. The results of this experiment are shown in Figure 1.

As indicated in Figure 1, at 300 days of age, 2 out of 17 mice (12%) in the FCA treated group had become diabetic while 12 out of 16 mice (75%) of their saline treated littermates became diabetic. At 300 days of age only 1 out of 14 (7%) of the QFA treated mice had developed diabetes compared to 8 out of 13 (62%) of their saline treated littermates.

### EXAMPLE 2

This example demonstrates the effectiveness of single 20 or 70 µl doses of QFA in inhibiting the occurrence of IDDM in NOD mice.

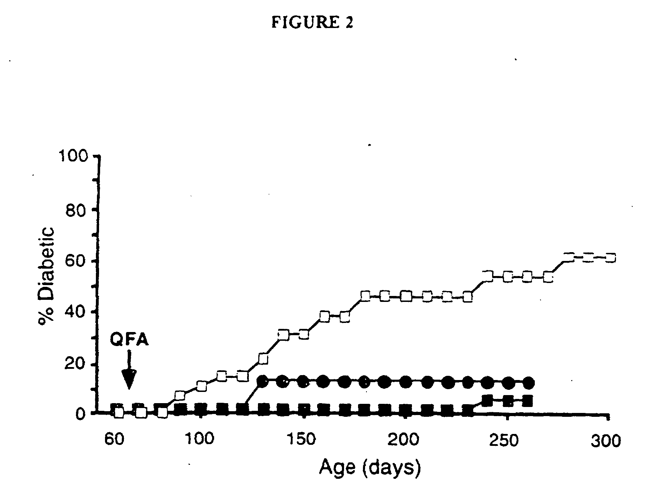

Female NOD mice between 62 and 72 days of age were injected intraperitoneally (ip) with either 70 µl of QFA or 20 µl of QFA. Fifteen mice were injected with 70 µl of QFA and 15 mice received 20 µl QFA. One animal from the 20 µl group was found dead of unknown causes during the course of the experiment. The 200 µl saline injected group of female NOD mice from Example 1 served as control animals. Mice were checked three times a week for elevated glucose levels in their urine (Tes-Tape, Eli Lilly and Co, Indianapolis, IN, USA). When positive for urinary glucose, whole blood glucose levels were determined with a Companion 2 sensor (Medisense, Cambridge, MA, USA). Where two consecutive daily blood glucose readings above the 95% confidence interval for NOD mice in this colony (3.9-9.1 mmol/L) were obtained, the animal was deemed to be diabetic. The animals were monitored until they reached 260 days of age or became diabetic. The results of this experiment are shown in Figure 2. At 260 days of age only 2 out of 15 (13%) of the group treated with 70 µl of QFA and 1 out of 14 (7%) of the 20 µl QFA treated group had become diabetic, compared to 8 out of 13 (62 %) of the saline treated NOD mice from Example 1 that served as controls for this experiment.

### EXAMPLE 3

This example demonstrates that QFA is more efficacious than either FCA or BCG in protecting beta cells from autoimmune destruction in NOD mice.

A group of 10 specific pathogen-free, 61- 66 day old female NOD mice were injected in both rear footpads with 40 µl (total dose 80 µl) reconstituted freeze-dried live BCG (Pasteur Merieux, Lyon, France). A second group of 17, 64-85 day old female NOD animals, were injected with FCA emulsified with an equal volume of normal saline (50 µl in right hind footpad). A third group of 14 age-matched female NOD mice were injected, ip, with 200 µl of QFA. Two additional groups of 14 age-matched female NOD mice received either 70 or 20 µl of QFA, ip. When the mice had reached 300 days of age, 10, 13 and 11 mice from the BCG, FCA and each of the QFA treated groups respectively (all nondiabetic) were sacrificed by cervical dislocation and their pancreases removed for histological sectioning and examination. Thirteen age-matched nondiabetic female NODs were also sacrificed and their pancreases similarly examined. Pancreases were fixed in 10% v/v neutral buffered formalin for 18 hours. Immunohistochemistry was used to stain for the presence of insulin and glucagon positive islets (DAKO Corporation, Carpinteria, CA). Serial 5 µm sections were stained for either insulin or glucagon and examined by light microscopy. As shown in Table 1 only 16% of islets in the BCG treated group stained positive for insulin production, 52 % of islets in the FCA treated group stained positive for insulin and in the QFA treated group 87%, 77% and 75% of islets stained positive for insulin production in the 200, 70 and 20 µl treatment groups respectively. Seventy-three percent of the islets examined from pancreases of age-matched female non-diabetic NOD mice had insulin positive islets while 27% of the islets were positive for glucagon only.

**TABLE 1**

| Treatment | % Insulin positive islets* | % Collapsed Islets * (Glucagon only) |
|---|---|---|
| **BCG** | **16** (11/69) | **84** (58/69) |
| **FCA** | **52** (31/60) | **48** (29/60) |
| **QFA (200 µl)** | **87** (105/121) | **13** (16/121) |
| **QFA (70 µl)** | **77** (40/52) | **23** (12/52) |
| **QFA (20 µl)** | **75** (41/55) | **25** (14/55) |
| **Age matched females** | **73** (22/30) | **27** (8/30) |

| | | |
|---|---|---|
| *Figures in parentheses represent the number of positive or collapsed islets/total number examined. | | |

### EXAMPLE 4

This example demonstrates the effectiveness of Q fever antigen treatment in preventing the recurrence of IDDM in spontaneously diabetic NOD mice transplanted with syngeneic islet tissue.

Donor animals, four to eight week old NOD female mice, were anaesthetised with an intraperitoneal injection of avertin solution. A curved 27 gauge needle was inserted into the common bile duct at the hilus after the distal end of the duct had been clamped. Approximately 3 ml of cold collagenase P (Boehringer Mannheim, Castle Hill, NSW, Australia) made to 2.5 mg/ml in Hanks balanced salt solution + 0.05% w/v BSA was injected immediately after dissection of the intrathoracic aorta. The pancreas was then excised and digested while held stationary in a 37°C water bath for 15 minutes and islets were hand-picked and transplanted essentially as described in Bowen *et al*.

Recipient mice, female NODs which had been diabetic for less than 2 weeks but for at least 3 days prior to transplantation and which had a mean blood glucose levels of 29.7 ± 1.38 mmol/L (range 21.2 - 33.4 mmol/L) on the day of engraftment, following anaesthesia (avertin, i.p.), received 450 islets under the left kidney capsule in a clot of recipient blood. These animals were divided into two groups. The treatment group, n=9, received QFA, 70 µl, s.c., at the time of transplantation and the control group, n=10 received an injection of saline, 70 µl, s.c. at the time of transplantation.

Blood glucose levels were determined for all islet-engrafted mice beginning on the day following transplantation. In the majority of cases in both the treated and control groups, a few days were required for normoglycaemia to be established. All 10 untreated islet-transplanted mice were hyperglycaemic (blood glucose > 22 mmol/L) by day 10 post-transplantation. Mice which were treated with QFA were largely protected against disease recurrence as evidenced by their blood glucose levels (mean blood glucose levels 8.17 ± 0.67) on day 10 post-transplantation. Thus, in the QFA-treated islet-transplanted group 6 out of 9 mice were protected from disease recurrence for greater than 30 days. One mouse out of 9 became diabetic on day 25 post-transplantation and two mice failed to become normoglycaemic following engraftment.

### EXAMPLE 5

This example demonstrates that *C. burnetii* vaccine (QVAX) is effective in inhibiting the occurrence of IDDM in NOD mice.

Female NOD littermates 72 days of age were divided into two groups and injected subcutaneously with either 100 µl of QVAX (obtained from CSL, Melbourne, Australia) or 100 µl of saline solution. Ten mice were treated with QVAX while 10 littermates received saline solution.

Mice in both groups were checked three times per week for elevated glucose concentrations in their urine (Tes-Tape, Eli Lilly and Co., Indianapolis, IN). When mice were found to have raised urinary glucose levels, whole blood glucose levels were determined with a Companion 2 Sensor (Medisense, Cambridge, MA, USA). Where two consecutive daily blood glucose readings above the 95 % confidence interval for NOD mice in this colony (3.9-9.1 mmol/L) were obtained, the animal was deemed to be diabetic. Animals were monitored until they reached 100 days of age or became diabetic.

None of the QVAX treated mice became diabetic during this period while 2 out of 10 of the saline treated mice became diabetic by 100 days of age. At 200 days of age, 9 out of 10 QVAX-treated mice were protected from developing diabetes while 9 out of 10 saline-treated mice developed diabetes.

### BIBLIOGRAPHY

Atkinson. M. A., et al *Diabetes*. **35**, 894-898, 1986.
Bach, J. F.. *Endocr*. *Rev*., **15**, 516-54, 1994.
Bowen, K. M., *et al Diabetes*, **29**, 98-104, 1980.
Davis, C. B., *et al J. Exp. Med*., **169**, 2239-2244, 1989.
Gross, D. J., *et al Diabetologia*, **37**, 1195-1201, 1994.
Harada, M., *et al Diabetes Res. Clin. Pract*., **8**, 85-89, 1990.
Honeyman, M.C. and Harrison, L.C. *Springer Semn. Immunopathol*., **14**, 253, 1993.
Kanazawa, Y. *et al Diabetologia*., **27,** 113-115, 1984.
Lakey, J. R., *et al Transplant. Proc*., **24**, 2848, 1992.
Lakey, J. R., *et al Transplantation*, **57**, 1213-1217, 1994.
Makino S. *et al Exp. Anim*., **29**, 1-13. 1980.
McInerney, M. F., *et al Diabetes*, **40**, 715-725, 1991.
Qin, H.-Y., *Autoimmunity*, **12**, 193-199, 1992.
Qin, H.-Y., *et al J. Immunol*. **150**, 2072-2080, 1993.
Sadelain, M. W. J., *et al Diabetes*, **39,** 583-589, 1990a.
Sadelain, M. W. J., *et al Journal of Autoimmunity*, **3**, 671-680, 1990b.
Shehadeh, N. N., *et al J. Autoimmun*., **6**, 291-300, 1993.
Toyota, T., *et al Diabetes*, **35,** 496-499, 1986.
Ulaeto, D., *et al Proc. Natl. Acad. Sci. (USA)*. **89**, 3927-3931, 1992.
Wang, T., *et al Diabetes*, **41,** 114-117, 1992.
Yagi, H., *et al Cell. Immunol*., **138**, 142-149, 1991.

## Claims

1. Use of a species of *Coxiella* which has been rendered non-infectious in the manufacture of a medicament for use in the treatment of an autoimmune disease in a mammal.

2. Use according to claim 1 wherein the mammal is a human or a laboratory test animal.

3. Use according to claim 1 wherein the autoimmune disease adversely effects the survival of pancreatic islet tissue transplanted into a mammal.

4. Use according to claim 2 wherein the autoimmune disease is IDDM.

5. Use of a species of *Coxiella* which has been rendered non-infectious in the manufacture of a medicament for use in prolonging survival of islet tissue transplanted into a mammal.

6. Use according to claim 5 wherein the mammal is a human or a laboratory test animal.

7. Use according to claim 2 or claim 5 wherein the species of *Coxiella* is *C. burnetii*.

8. Use according to claim 2 or claim 5 wherein the *C*. *burnetii* is in the form of a killed preparation.

9. Use according to claim 7 or claim 8 wherein the *C*. *burnetii* is in the form of a whole cell preparation.

10. Use according to claim 9 wherein the antigenic component from *C*. *burnetii* is Q fever complement fixing antigen phase I (QFA).

11. Use according to any of claims 7 to 10 wherein the *C. burnetii* is in the form of the preparation, QVAX.

12. Use according to any of claims 1 to 8 wherein the *C*. *burnetii* is in the form of a lysed preparation of the whole organism, a membrane/wall preparation or an endospore preparation.

## Patentansprüche

1. Verwendung einer Spezies von *Coxiella*, die nicht infektiös gemacht worden ist, zur Herstellung eines Arzneimittels zur Verwendung in der Behandlung einer Autoimmunkrankheit in einem Säuger.

2. Verwendung nach Anspruch 1, worin der Säuger ein Mensch oder ein zu Testzwecken im Labor eingesetztes Tier ist.

3. Verwendung nach Anspruch 1, worin die Autoimmunkrankheit das Überleben von Inselzellengewebe aus dem Pankreas, das in einen Säuger transplantiert wurde, beeinträchtigt.

4. Verwendung nach Anspruch 2, worin die Autoimmunkrankheit IDDM ist.

5. Verwendung einer Spezies von *Coxiella*, die nicht infektiös gemacht worden ist, zur Herstellung eines Medikaments zur Verwendung in der Verlängerung des Überlebens von Inselzellengewebe, das in einen Säuger transplantiert worden ist.

6. Verwendung nach Anspruch 5, worin der Säuger ein Mensch ist oder ein zu Testzwecken im Labor eingesetztes Tier.

7. Verwendung nach Anspruch 2 oder 5, worin die Spezies von *Coxiella C. burnetii* ist.

8. Verwendung nach Anspruch 2 oder 5, worin *C. burnetii* in Form einer getöteten Vorbereitung vorliegt.

9. Verwendung nach Anspruch 7 oder 8, worin *C. burnetii* in Form einer Gesamtzell-Zubereitung vorliegt.

10. Verwendung nach Anspruch 9, worin der antigene Bestandteil von *C. burnetii* die Q-Fieber komplementfixierende Phase QFA ist.

11. Verwendung nach einem der Ansprüche 7 bis 10, worin *C. burnetii* in Form der Zubereitung QVAX vorliegt.

12. Verwendung nach einem der Ansprüche 1 bis 8, worin *C. burnetii in* der Form einer lysierten Zubereitung des gesamten Organismus, einer Membranwandzubereitung oder einer Endosporenzzubereitung vorliegt.

## Revendications

1. Utilisation d'une espèce de *Coxiella* qui a été rendue non infectieuse dans la fabrication d'un médicament pour utilisation dans le traitement d'une maladie auto-immune chez un mammifère.

2. Utilisation selon la revendication 1 dans laquelle le mammifère est un humain ou un animal de test de laboratoire.

3. Utilisation selon la revendication 1 dans laquelle la maladie auto-immune affecte négativement la survie d'un tissu d'îlots pancréatiques transplanté chez un mammifère.

4. Utilisation selon la revendication 2, dans laquelle la maladie auto-immune est le diabète mellitus insulino-dépendant.

5. Utilisation d'une espèce de *Coxiella* qui a été rendue non infectieuse dans la fabrication d'une médicament pour utilisation dans la prolongation de la survie d'un tissu d'îlots transplanté chez un mammifère.

6. Utilisation selon la revendication 5 dans laquelle le mammifère est un humain ou un animal de test de laboratoire.

7. Utilisation selon la revendication 2 ou la revendication 5 dans laquelle l'espèce de *Coxiella* est *C. burnetii*.

8. Utilisation selon la revendication 2 ou la revendication 5 dans laquelle *C. burnetii* est sous la forme d'une préparation tuée.

9. Utilisation selon la revendication 7 ou la revendication 8 dans laquelle *C. burnetii* est sous la forme d'une préparation de cellules entières.

10. Utilisation selon la revendication 9 dans laquelle le composant antigénique de *C. burnetii* est l'antigène de phase I de la fièvre Q (QFA) fixant le complément.

11. Utilisation selon l'une quelconque des revendications 7 à 10 dans laquelle *C. burnetii* est sous la forme de la préparation QVAX.

12. Utilisation selon l'une quelconque des revendications 1 à 8 dans laquelle *C. burnetii* est sous la forme d'une préparation lysée de l'organisme entier, d'une préparation de membrane/paroi ou d'une préparation d'endospore.
